(19) Europäisches Patentamt
European Patent Office
Office européen des brevets

(11) **EP 2 825 152 B1**

(12) **FASCICULE DE BREVET EUROPEEN**

(45) Date de publication et mention de la délivrance du brevet:
**08.06.2016 Bulletin 2016/23**

(51) Int Cl.:
***A61K 8/97*** *(2006.01)*    ***A61K 36/18*** *(2006.01)*
***A61Q 5/00*** *(2006.01)*

(21) Numéro de dépôt: **13709245.8**

(22) Date de dépôt: **15.03.2013**

(86) Numéro de dépôt international:
**PCT/EP2013/055426**

(87) Numéro de publication internationale:
**WO 2013/135875 (19.09.2013 Gazette 2013/38)**

(54) **UTILISATION COSMETIQUE D'UN EXTRAIT D'AMANDES DE BALANITES POUR AMELIORER LA RESISTANCE DES CHEVEUX**

KOSMETISCHE VERWENDUNG EINES EXTRAKTES AUS BALANITES-MANDELN ZUR VERBESSERUNG DER FESTIGKEIT VON HAAR

COSMETIC USE OF AN EXTRACT OF BALANITES ALMONDS TO IMPROVE HAIR STRENGTH

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **15.03.2012 FR 1252337**

(43) Date de publication de la demande:
**21.01.2015 Bulletin 2015/04**

(73) Titulaire: **Pierre Fabre Dermo-Cosmétique**
**92100 Boulogne-Billancourt (FR)**

(72) Inventeur: **FIORINI PUYBARET, Christel**
**F-31500 Toulouse (FR)**

(74) Mandataire: **Regimbeau**
**20, rue de Chazelles**
**75847 Paris Cedex 17 (FR)**

(56) Documents cités:
**FR-A1- 2 962 907**

- **DATABASE GNPD [Online] MINTEL; 1 juin 2007 (2007-06-01), ANONYMOUS: "Emulsion color", XP002686853, extrait de www.gnpd.com Database accession no. 717807**
- **E.A. ABDEL-RAHIM, S.S. EL-SAADANY, M.M. WASIF: "Biochemical dynamics of hypocholesterolemic action of Balanites aegyptiaca fruit", FOOD CHEMISTRY, vol. 20, 1 janvier 1986 (1986-01-01), pages 69-78, XP002686854,**

- **CHAPAGAIN BISHNU ET AL: "Variation in diosgenin level in seed kernels among different provenances of Balanites aegyptiaca Del (Zygophyllaceae) and its correlation with oil content", AFRICAN JOURNAL OF BIOTECHNOLOGY, vol. 4, no. 11 1 novembre 2005 (2005-11-01), pages 1209-1213, XP008157777, ACADEMIC PRESS, US ISSN: 1684-5315 Extrait de l'Internet: URL:http://www.academicjournals.org/AJB/index.htm**
- **KUSCH PETER ET AL: "In Vitro and In Vivo Antimalarial Activity Assays of Seeds from Balanites aegyptiaca: Compounds of the Extract Show Growth Inhibition and Activity against Plasmodial Aminopeptidase.", JOURNAL OF PARASITOLOGY RESEARCH, vol. 2011, 368692, mars 2011 (2011-03), pages 1-9, XP002686855, ISSN: 2090-0031**
- **DATABASE GNPD [Online] MINTEL; 1 février 2012 (2012-02-01), ANONYMOUS: "Leave-in combing cream", XP002686856, extrait de www.gnpd.com Database accession no. 1737547**
- **AL ASHAAL H A ET AL: "Phytochemical investigation and medicinal evaluation of fixed oil of Balanites aegyptiaca fruits (Balantiaceae)", JOURNAL OF ETHNOPHARMACOLOGY, vol. 127, no. 2, 3 février 2010 (2010-02-03), pages 495-501, XP026853075, ISSN: 0378-8741 [extrait le 2009-10-13]**

**(Cont. page suivante)**

• DATABASE GNPD [Online] MINTEL; 1 juin 2009 (2009-06-01), ANONYMOUS: "Intense moisturizing oil", XP002686857, extrait de www.gnpd.com Database accession no. 1121583

• DATABASE GNPD [Online] MINTEL; 1 octobre 2012 (2012-10-01), ANONYMOUS: "Shampoo", XP002686858, extrait de www.gnpd.com Database accession no. 1909942

**Description**

La présente invention concerne de façon générale les compositions capillaires et plus particulièrement des compositions destinées à améliorer la résistance des cheveux par une action anti-casse.

Le domaine de la présente invention concerne une nouvelle valorisation d'un extrait d'amandes d'un végétal appartenant au genre Balanites de la famille des Zygophyllaceae. Préférentiellement la présente invention concerne les travaux menés sur l'espèce *Balanites aegyptiaca* L. Delile en raison de la facilité de son approvisionnement en Afrique. Mais cette caractéristique n'est pas limitative et un extrait de qualité comparable avec d'autres espèces du genre Balanites provenant d'autres pays peut aussi être envisagé. On peut citer par exemple : *Balanites triflora Tiegh et Balanites roxburgghii Planch.*

Le Balanites *aegyptiaca* est aussi plus communément appelé Dattier du désert.

*Balanites* est un genre tropical afro-asiatique, de 25 espèces environ, des régions sèches subarides et même arides à pluviométries moyennes annuelles comprises entre 1 000 mm et 200 mm et même 100 mm. Son aire de distribution géographique s'étend de l'Afrique, à l'Ouest (côtes sénégalo-mauritaniennes) jusqu'en Birmanie, à l'Est. *Balanites aegyptiaca* est présent dans l'Afrique tropicale sèche, du Sénégal au Soudan, l'Afrique Orientale, de l'Egypte à la Zambie, l'Arabie et l'Inde. Il est arrivé en Asie depuis la Méditerranée via l'Égypte. C'est un des arbres les plus communs au Sénégal.

Le dattier du désert est un arbre à cime sphérique, aplatie ou irrégulière atteignant 8 à 9 m de haut. Port remarquable avec ses branches retombantes souples, armées de longues épines alternes ou disposées plus ou moins en spirale.

Son fruit est une drupe ellipsoïde de 5 x 2,5 cm, verte et pubescente, devenant jaune et plus ou moins glabre à maturité. Une peau mince enveloppe une pulpe comestible (qui représente 43% du poids du fruit frais) autour d'un noyau dur, ovoïde et pointu : contenant l'amande.

La richesse du fruit en glucides et vitamines justifie son nom de « Datte du désert », il est d'ailleurs très apprécié des enfants et des adultes.

Toutes les parties de l'arbre sont utilisées dans la pharmacopée traditionnelle africaine.

Racines : Morsure de serpent, charbon

Ecorce : Ictère, fièvre jaune, syphilis, toux, épilepsie et anxiété.

Epines : Lèpre

Feuilles : Asthénie, charbon

Fruits : Rhumatismes. Laxatif doux

Graines : Huile employée en pommade et onguent avec de nombreux médicaments. L'huile d'amande est utilisée comme corps gras pour la fabrication artisanale de savons.

L'état de la technique antérieure dans le domaine cosmétique concerne principalement l'huile de Balanites (on peut citer EP 0 781 545 - axe hydratation des couches supérieures de la peau).

De façon surprenante et inattendue, la demanderesse a mis en évidence une activité originale d'un extrait d'amandes de Balanite tel que défini dans les revendications, sur le pouvoir anti-casse des cheveux. Ce type d'activité est particulièrement intéressant pour améliorer la résistance des cheveux et plus particulièrement des cheveux secs, abîmés et cassants.

Par la suite, la teneur des composés de l'extrait sera exprimée en extrait sans support de séchage.

L'extrait d'amandes de Balanites selon la présente invention est caractérisé en ce qu'il comprend une teneur en cires, acides aminés, protéines et sucres totaux.

Au sens de la présente invention, par « cires » on entend les esters d'alcool gras ; et par « sucres totaux » on entend la teneur en sucre après hydrolyse de l'extrait.

Qualitativement, l'extrait selon la présente invention se différencie fortement de l'huile. En effet, bien que certains acides gras et hydrocarbures soient aussi présents dans notre extrait comme dans l'huile ; en revanche les cires, acides aminés libres, protéines et sucres sont absents de l'huile. Ce sont les caractéristiques techniques retenues pour différencier notre extrait d'une huile de Balanites déjà utilisée en cosmétique.

L'extrait utile dans le cadre de la présente invention peut être préparé comme suit.

Les amandes des graines du Dattier du désert sont de préférence extraites par un mélange d'eau et d'un ou plusieurs solvants organiques.

Le solvant organique peut être un alcool (méthanol, éthanol, propanol, isopropanol, butanol, octanol), une cétone (méthyléthycétone, méthylisobutylcétone), ainsi que leurs mélanges.

L'extrait sans support de séchage est obtenu avec rendement de 25 % environ. Cet extrait est hétérogène, constitué d'une phase grasse liquide (phase supérieure, 65 %) et d'une phase solide à l'aspect d'un beurre (phase inférieure, 35 %). L'ajout d'un support inerte (tel que maltodextrine) permet d'obtenir un extrait homogène.

L'extrait peut également être stabilisé par addition d'un antioxydant comme par exemple le butylhydroxytoluène ou de l'alpha tocophérol à des quantités comprises entre 0,05 à 1 g%g d'extrait sec.

Caractérisation de l'extrait (teneurs exprimées g / 100 g d'extrait sans support de séchage) :

- cires : de 1 % à 10 % et préférentiellement 4%
- acides aminés libres : 1% à 5 % et préférentiellement 2%
- protéines : de 0,5 % à 10 % et préférentiellement de 1 à 5%
- sucres totaux : de 20 % à 60 % et préférentiellement 28%.

La présente invention concerne l'utilisation d'une composition cosmétique pour améliorer la résistance des cheveux, ladite composition cosmétique comprenant à titre de principe actif un extrait d'amandes de Balanites tel que décrit précédemment.

Cet extrait de Balanites est de plus, compte tenu de sa composition chimique riche en sucres, en acides aminés et protéines, en cires et en acides gras, particulièrement nutritif vis-à-vis des cheveux.

L'extrait d'amandes de Balanites selon la présente invention permet de nourrir la fibre capillaire en lui apportant les éléments essentiels à sa résistance et sa nutrition et confère aux cheveux traités une meilleure résistance à la cassure.

Préférentiellement, les cheveux traités sont fragilisés, secs, abimés et cassants.

Il a été observé une augmentation significative de la contrainte à la cassure pour les cheveux traités à l'aide d'un extrait selon l'invention.

La présente invention concerne enfin une méthode de traitement cosmétique des cheveux destiné à améliorer la résistance des cheveux par un effet anti-casse consistant en l'application sur les cheveux d'une composition cosmétique comprenant un extrait d'amandes de Balanites selon l'invention.

De préférence, la quantité d'extrait d'amandes de Balanites est comprise entre 0,05 et 5 % par rapport au poids total de la composition et de préférence, ladite quantité d'extrait est comprise entre 0,05 % et 1 % par rapport au poids total de la composition.

La composition cosmétique selon la présente invention peut avantageusement se présenter sous toutes les formes galéniques normalement utilisées dans le domaine cosmétique capillaire pour une application topique. Préférentiellement, la forme topique peut être notamment sous forme de: shampoing, baume, masque, gel, lotion, mousse, spray, crème.

On distingue ainsi des produits formulés pour être rincés et d'autres non.

La composition cosmétique selon l'invention comprend, en outre, des excipients usuels cosmétiquement compatibles.

Les excipients usuels compatibles à la composition cosmétique capillaire peuvent être tout excipient parmi ceux connus de l'homme du métier en vue d'obtenir une composition cosmétique pour une application topique sous les formes telles que décrites ci-dessus.

La composition cosmétique selon l'invention peut en particulier contenir des additifs et aides à la formulation, tels que des tensio-actifs de type émulsionnant, nettoyant, moussant, etc. des agents complexants, des épaississants, des gélifiants, des stabilisants, des conservateurs dont des antimicrobiens et des antioxydants, des conditionneurs, des acidifiants, des alcalinisants, des émollients, des solvants, des colorants, des parfums.

Les exemples suivants sont donnés à titre indicatif non limitatif.

Exemple 1 de préparation de l'extrait végétal selon l'invention :

*1 kg d'amandons de Balanites sont extraits par 10 litres d'alcool* à 80 % *(v/v) à reflux pendant 1 heure sous agitation.*

Après filtration et rinçage du marc, le filtrat est concentré puis séché sur 750 g de la maltodextrine puis stabilisé par addition de 0,2 % d'alpha-tocophérol.

L'extrait est une poudre jaunâtre obtenu avec un rendement voisin de 100 % (p/p).
L'extrait renferme (valeur exprimée sans support de séchage) : 4 % de cires, 2,1 % d'acides aminés libres, 1,1 à 5 % de protéines et 28% de sucres totaux.

Exemple 2 : *1 kg d'amandons de Balanites sont extraits par 10 litres d'alcool (v/v) à température ambiante pendant 2 heures sous agitation. Après filtration et rinçage du marc, le filtrat est concentré jusqu'à 2 litres (concentrât 1) et stabilisé par addition de Butylhydroxytoluène (B.H.T.).*

Le marc épuisé à l'alcool est extrait à par 10 litres d'EtOH 80 % (v/v). Après filtration et rinçage du marc, le filtrat est concentré jusqu'à 2 litres (concentrât 2).

A 0,8 litre de concentrât 2 est mélangé 0,2 litre de concentrât 1. Après addition de 750 g de maltodextrine, l'ensemble est séché au rotavapor.

L'extrait a une composition similaire à celle de l'extrait de l'exemple 1.

Exemples de compositions cosmétiques :

Exemple 3 : Baume à rincer

| | |
|---|---|
| EXTRAIT AMANDES BALANITES | 0.1 % |
| GUAR HYDROXYPROPYLTRIMONI.CHL. | 0.3 % |
| PROPYLENE GLYCOL | 0.5 % |
| SCLEROTIUM GUM | 0.3 % |
| CETEARYL ALC./CETEARYL MEL. | 2 % |
| INULIN LAURYL CARBAMATE | 0.25 % |
| CETYLIQUE ALCOOL | 7 % |
| PALMITATE ETHYLHEXYL | 8 % |
| DICAPRYLYL CARBONATE | 2 % |
| PROTEINE BLE HYDROLYSE | 2 % |
| SORBIQUE ACIDE | 0.3 % |
| SALICYLIQUE ACIDE | 0.15 % |
| PHENOXYETHANOL | 0.7 % |
| COLORANTS | QSP |
| PARFUM | QSP |
| HYDROXYDE SODIUM | QSP |
| EAU PURIFIEE | QSP |

Exemple 4 : Shampooing

| | |
|---|---|
| EXTRAIT AMANDES BALANITES | 0.1 % |
| PROTEINE BLE HYDROLYSE | 0.5 % |
| COCOATE GLYCERYL PEG-7 | 8 % |
| HUILE OLIVE ETHOXYLEE | 3 % |
| QUATERNIUM(POLY)10 | 0.5% |
| DECYL GLUCOSIDE | 2 % |
| NA LAUROYL METHYL ISETHIONATE | 2 % |
| LAURETH SULFATE SODIUM | 13 % |
| BETAINE LAURYL | 5 % |
| BENZOATE SODIUM | 0.4 % |
| EDTA SODIUM(DI) | 0.2 % |
| COLORANTS | 0.1 % |
| BASE NACRANTE | 8 % |
| PARFUM | QSP |
| CITRIQUE ACIDE MONOHYDRATE | QSP |
| CHLORURE SODIUM DESULFATE | QSP |
| EAU PURIFIEE | QSP |

Exemple 5 : Masque

| | |
|---|---|
| PROTEINE BLE HYDROLYSE | 3 % |
| EXTRAIT AMANDES BALANITES | 0.2% |
| SALICYLIQUE ACIDE | 0.15% |

(suite)

| | |
|---|---|
| GUAR HYDROXYPROPYLTRIMONI.CHL. | 0.5 % |
| PROPYLENE GLYCOL | 1 % |
| BEHENTRIMONIUM CHLORURE | 3 % |
| CETEARYL ALC./CETEARYL MEL. | 1 % |
| INULIN LAURYL CARBAMATE | 0.15 % |
| PALMITATE GLYCOL | 6 % |
| BEHENIQUE ALCOOL | 4 % |
| ADIPATE DIISOPROPYL | 10 % |
| HUILE RICIN | 3 % |
| PHENOXYETHANOL | 0.7 % |
| SORBIQUE ACIDE | 0.3 % |
| PARFUM | QSP |
| LACTIQUE ACIDE | QSP |
| BUTYLHYDROXYTOLUENE | 0.05 % |
| COLORANTS | QSP |
| EAU PURIFIEE | QSP |

Exemple 6 : Soin sans rinçage

| | |
|---|---|
| EXTRAIT AMANDES BALANITES | 0.1 % |
| PROTEINE BLE HYDROLYSE | 0.1 % |
| ACRYLATE(POLY)13 MEL. | 1.5 % |
| CAPRYLYL(DI)ETHER | 5 % |
| HUILE RICIN | 5 % |
| ALKYL C14-22 ALCOOL MEL. | 1.25 % |
| GUAR HYDROXYPROPYLTRIMONI.CHL. | 0.3 % |
| PROPYLENE GLYCOL | 0.5 % |
| PHENOXYETHANOL | 0.5 % |
| SORBIQUE ACIDE | 0.2 % |
| BUTYLHYDROXYTOLUENE | 0.01 % |
| PARFUM | QSP |
| COLORANT | QSP |
| EAU PURIFIEE | QSP |

Exemple 7 : Mesures des propriétés mécaniques du cheveu traité par un extrait d'amandes de Balanites aegyptiaca.

Les propriétés mécaniques du cheveu sont mesurées à l'aide d'un extensomètre linéaire qui applique une traction jusqu'à la cassure du cheveu. Ainsi, grâce à un capteur de force, la force de cassure peut être mesurée. En tenant compte de la section du cheveu, la force de cassure est ramenée à la section et exprimée comme la contrainte à la cassure.

METHODE

- Cheveux utilisés

Cheveux d'origine : Euro-nature, brun, glued weft dense, couleur 5/0, longueur totale 20cm dont 18cm libre. Fourni par la société Kerling Intl (Allemagne).

Et cheveux fragilisés obtenus par traitements chimiques des cheveux d'origine : Décoloration par oxydation chimique pendant 4h suivi par une permanente de 30 mn.

La mèche de cheveux fragilisés a été découpée en plusieurs sous-mèches d'une largeur de 0,5cm (poids moyen = 270 ± 30mg). Par la suite, ces sous-mèches ont fait l'objet de l'essai, en raison d'un produit testé par sous-mèche.

- Produits testés

Les préparations testées ont été obtenues en mettant respectivement 5 g et 0,5 g d'extrait préparé selon l'exemple 1 en suspension dans de l'éthanol à 27%.

➢ 100mL d'extrait de dattier à 5% dans de l'éthanol à 27%.

➢ 100mol d'extrait de dattier à 0,5% dans de l'éthanol à 27%.

➢ 100mL d'éthanol à 27%.

- Application des produits

Les cheveux fragilisés ont été traités par les préparations en laissant tremper les mèches pendant 96 heures dans les solutions, et en les agitant pendant ce temps.

Les mèches de cheveux fragilisés non traitées ont servi comme témoin.

A la fin du traitement, les mèches de cheveux ont étés lavées pendant 30 secondes avec du lauryl sulfate de sodium à 3%, suivi d'un rinçage à l'eau claire pendant 1 minute.

Enfin, les mèches ont séchées pendant 24h à l'air libre avant de servir aux tests selon le protocole ci-dessous.

MESURE DES PROPRIETES MECANIQUES

L'appareillage utilisé est un Miniature Tensile Tester 675 (MTT, Diastron, UK) associé avec un Laser Scan Micrometer (LSM, Mitutoyu, Japon). La procédure de mesure et d'analyse des cheveux est comme suit :

➢ Sertissage des cheveux individuels avec une longueur de test de 30 mm (n=40 cheveux par traitement).

➢ Mesure de la section de chaque cheveu par le LSM.

➢ Traction des cheveux mouillés (1 h de trempage dans l'eau) jusqu'à la cassure par le MTT. Vitesse de traction constante = 10 mm/mn

➢ Analyse de la courbe de traction afin d'identifier et relever la contrainte de la cassure en megapascal (MPa, où 1 Pa = 1N/m$^2$), qui indique la force de cassure pondérée par la section du cheveu. Ainsi, on s'affranchit de la variabilité de l'épaisseur des cheveux étudiés.

Cette procédure a été utilisée pour mesurer et analyser des mèches ayant été traitées et également les mèches n'ayant reçu aucun traitement (témoins non-traités).

# RESULTATS (n= 40 cheveux par traitement)

Les résultats des mesures de la contrainte à la cassure sont présentés dans le tableau 1, avec les valeurs moyennes des contraintes à la cassure et les écarts types correspondants. Les différences par rapport au témoin non-traité ont été calculées sous forme de pourcentages.

Tableau 1 : Contrainte à la cassure en MPa pour les cheveux testés en fonction des traitements.

| Traitement | Moyenne de la contrainte à la cassure (MPa) | *Ecart-type* | % |
|---|---|---|---|
| Témoin Non Traité | 51.56 | *11.46* | 0.0 |
| 0,5% dattier | 55.97 | *7.59* | 8.5 |
| 5% dattier | 57.67 | *10.00* | 11.8 |

Une analyse statistique (Student T test, non-apparié, bilatéral) de ces résultats a été réalisée avec le test de Student (seuil 5%)

➢ Traité avec 0,5% extrait de Balanites, p = 0,0468 (Significatif)

➢ Traité avec 5% extrait de Balanites, p = 0,0131 (Significatif)

CONCLUSIONS

Dans ces conditions expérimentales, on observe une augmentation significative de la contrainte à la cassure pour les deux concentrations d'extraits de dattier par rapport au témoin non-traité.

Ces résultats traduisent un effet renforçant de l'actif sur les cheveux fragilisés. Ainsi, ces derniers, lorsqu'ils ont été traités par l'extrait et soumis à une traction à vitesse constante, se cassent à une force plus importante que celle des cheveux non-traités.

Cette étude a permis de démontrer l'efficacité d'une composition selon la présente invention sur le pouvoir anti-casse des cheveux.

**Revendications**

1. Utilisation d'une composition cosmétique pour améliorer la résistance des cheveux, ladite composition cosmétique comprenant à titre de principe actif un extrait d'amandes de Balanites comprenant les teneurs suivantes exprimées en pourcentages massiques par rapport à l'extrait sans support de séchage:

   - cires : de 1 % à 10% et préférentiellement 4%
   - acides aminés libres : de 1% à 5% et préférentiellement 2%
   - protéines : de 0,5% à 10% et préférentiellement de 1% à 5%
   - sucres totaux : de 20 à 60% et préférentiellement 28%.

2. Utilisation selon la revendication 1, **caractérisée en ce que** la quantité d'extrait est comprise entre 0,05 % et 5 % en poids par rapport au poids total de la composition.

3. Utilisation selon l'une des revendications 1 et 2, **caractérisée en ce que** ledit extrait est un extrait de *Balanites aegyptiaca, Balanites triflora Tiegh ou Balanites roxburgghii Planch* et préférentiellement *Balanites aegyptiaca.*

4. Méthode de traitement cosmétique des cheveux destinée à améliorer la résistance des cheveux par un effet anti-casse consistant en l'application sur les cheveux d'une composition cosmétique telle que définie à l'une des revendications 1 à 3.

**Patentansprüche**

1. Verwendung einer kosmetischen Zusammensetzung, um die Widerstandskraft der Haare zu verbessern, wobei die kosmetische Zusammensetzung als Wirkstoff einen Extrakt aus Balanites-Mandeln umfasst, umfassend die folgenden Gehalte, ausgedrückt in Gewichtsprozent mit Bezug auf den Extrakt ohne Trocknungsträger:

   - Wachse: von 1 % bis 10 % und vorzugsweise 4 %
   - Freie Aminosäuren: von 1 % bis 5 % und vorzugsweise 2 %
   - Proteine: von 0,5 % bis 10 % und vorzugsweise von 1 % bis 5 %
   - Gesamtzucker: von 20 bis 60 % und vorzugsweise 28 %.

2. Verwendung nach Anspruch 1, **dadurch gekennzeichnet, dass** die Menge des Extrakts zwischen 0,05 Gew% und 5 Gew% mit Bezug auf das Gesamtgewicht der Zusammensetzung liegt.

3. Verwendung nach einem der Ansprüche 1 und 2, **dadurch gekennzeichnet, dass** der Extrakt ein Extrakt von *Balanites aegyptiaca, Balanites triflora Tiegh* oder *Balanites roxburgghii Planch* und vorzugsweise *Balanites aegyptiaca* ist.

4. Verfahren zur kosmetischen Behandlung der Haare, das ausgelegt ist, um die Widerstandskraft der Haare durch eine Anti-Bruchwirkung zu verbessern, das darin besteht, auf die Haare eine kosmetische Zusammensetzung wie in einem der Ansprüche 1 bis 3 definiert, anzuwenden.

**Claims**

1. Use of a cosmetic composition for enhancing hair strength, said cosmetic composition comprising as an active substance a Balanites kernel extract comprising the following contents expressed as mass percentages with respect to the extract without drying substrate:

   - waxes: from 1% to 10% and preferentially 4%
   - free amino acids: from 1% to 5% and preferentially 2%
   - proteins: from 0.5% to 10% and preferentially from 1% to 5%
   - total sugars: from 20 to 60% and preferentially 28%.

2. Use according to claim 1, **characterised in that** the quantity of extract is between 0.05% and 5% by weight with respect to the total weight of the composition.

3. Use according to any one of claims 1 and 2, **characterised in that** said extract is an extract of *Balanites aegyptiaca, Balanites triflora Tiegh* or *Balanites roxburgghii Planch* and preferentially *Balanites aegyptiaca.*

4. Cosmetic treatment method for hair intended to enhance hair strength with an anti-breakage effect consisting of applying a cosmetic composition as defined in any one of claims 1 to 3 on the hair.

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 0781545 A